# DEMANDE DE BREVET EUROPEEN

(11) **EP 1 693 446 A1**
(43) Date de publication de la demande: **23.08.2006**
(21) Numéro de dépôt: 05447032.3
(22) Date de dépôt: 16.02.2005
(51) Int. Cl.: C12N 1/26, C02F 3/30

(54) **Composition - substrat pour microorganismes denitrifiants**

(71) Demandeur: Realco, 1348 Louvain-la-Neuve (BE)
(72) Inventeur: Boels, Gauthier, 1070 Bruxelles (BE); Blackman, Gordon, 1380 Lasne (BE); Demyttenaere, Philippe, 1350 Jauche (BE)
(74) Mandataire: Van Malderen, Joëlle

(57) **Abrégé**

L'invention se rapporte à une composition riche en acides gras utilisée comme substrat pour des microorganismes dénitrifiants, notamment dans le traitement des eaux usées.

L'invention se rapporte également à un procédé de préparation d'une composition riche en acides gras utilisée comme substrat pour des microorganismes dénitrifiants.

L'invention se rapporte également à un nouveau procédé de dénitrification et à un nouveau procédé de traitement des eaux usées.

## Description

### Domaine de l'invention

L'invention porte sur une composition riche en acides gras à longues chaînes carbonées, utilisée comme substrat pour des microorganismes dénitrifiants, notamment dans le traitement des eaux usées.

L'invention porte également sur un procédé de préparation d'une composition riche en acides gras à longues chaînes carbonées, substrat pour microorganismes dénitrifiants.

L'invention porte également sur un nouveau procédé de dénitrification et sur un nouveau procédé de traitement des eaux usées.

### Art antérieur

En station d'épuration (STEP), l'élimination de l'azote des eaux usées se fait par des microorganismes, par un processus de nitrification - dénitrification. La nitrification est une phase aérobie au cours de laquelle l'azote présent dans l'eau sous forme de NH₄ est oxydé en nitrate, puis transformé, au cours de la phase anaérobie de dénitrification en nitrite avant d'être éliminé sous forme d'azote gazeux (retour à l'atmosphère).

Cette étape de dénitrification nécessite l'apport de carbone sous une forme assimilable par les microorganismes dénitrifiants.

La source de carbone peut être l'effluent brut lui-même, mais dans nombre de cas, les quantités de carbone organique assimilable apportées par l'effluent sont insuffisantes, le recours à une source de carbone extérieure s'impose alors et consiste généralement en du méthanol, de l'éthanol ou plus communément en de l'acétate.

Il existe de nombreuses publications relatant les travaux portant sur la recherche d'alternatives au méthanol, à l'éthanol ou à l'acétate.

Ainsi, M. Mailloux et al. ont testé différents substrats, mais concluent que l'éthanol et l'acétate restent les meilleurs substrats pour les microorganismes dénitrifiants, confirmant les études déjà réalisées (Ground and water : theory to practice, 2002, Oct., 919-926, « Evaluation of a carbon-source stimulated biomediation technology for the remediation of a nitrate-contaminated aquifer at an airport site »).

Suite à ces nombreux travaux à la conclusion invariable, il était admis que l'éthanol ou l'acétate donnaient des cinétiques de réduction des nitrates supérieures aux autres substrats parce qu'ils étaient facilement biodégradables.

### Résumé de l'invention

Les inventeurs ont trouvé, contre toute attente, qu'une composition riche en acides gras à longues chaînes carbonées utilisée comme substrat pour des microorganismes dénitrifiants, donnait une cinétique de réduction des nitrates tout à fait comparable à celle obtenue avec de l'acétate.

Un objet de la présente invention est l'utilisation d'une composition comprenant des acides gras à longues chaînes carbonées (C12 à C20), comme substrat pour des microorganismes dénitrifiants.

Un objet de la présente invention est l'utilisation comme substrat pour des microorganismes dénitrifiants d'une composition comprenant des acides gras à longues chaînes carbonées (C12 à C20), notamment l'acide palmitique, l'acide stéarique et/ou l'acide oléique.

La présente invention concerne plus particulièrement l'utilisation d'une composition comprenant des acides gras dont plus de 60% sont en C12, C13, C14, C15, C16, C17, C18, C19 et/ou C20, comme substrat pour microorganismes dénitrifiants.

La présente invention concerne également l'utilisation d'une composition comme substrat pour microorganismes dénitrifiants comprenant 20% à 40% d'acide palmitique, 30% à 60% d'acide stéarique, 5% à 25% d'acide oléique, optionnellement jusqu'à 10% d'acide myristique et/ou optionnellement jusqu'à 25% d'acide arachidique par rapport à la totalité des acides gras présents dans la composition.

La concentration en acides gras (en poids) d'une composition selon l'invention peut varier de 0,01% à 100% par rapport au poids total de ladite composition, en passant par toutes les valeurs possibles entre ces deux extrêmes.

De façon avantageuse, les acide gras présents dans la composition représentent plus de 30% environ du poids total des MEH (matières extractibles à l'hexane), de préférence plus de 50%, 60%, ou plus de 70% environ, voire plus de 80%, 90%, 95%, 96%, 97% environ du poids total des MEH.

Un objet de la présente invention est une composition pour microorganismes dénitrifiants comprenant 20% à 40% d'acide palmitique, 30% à 60% d'acide stéarique, et/ou 5% à 25% d'acide oléique, optionnellement jusqu'à 10% d'acide myristique et/ou optionnellement jusqu'à 25% d'acide arachidique par rapport à la totalité des acides gras présents dans la composition.

La présente invention concerne également un procédé de dénitrification (nitrification - dénitrification) comprenant une étape de mise en contact, dans un milieu azoté, de microorganismes dénitrifiants et de substrat consistant en une composition comprenant des acides gras dont plus de 60% sont en C12, C13, C14, C15, C16, C17, C18, C19 et/ou C20 pour lesdits microorganismes dénitrifiants.

Un procédé de dénitrification de l'invention peut s'effectuer dans un tout bassin ou réacteur de traitement des eaux usées (anaérobie, aéré ou non), tels que des aérateurs, digesteurs, décanteurs, etc., contenant ledit milieu azoté et lesdits microorganismes dénitrifiants.

La présente invention concerne également un procédé de traitement des eaux usées domestiques ou industrielles comprenant une étape de dénitrification au cours de laquelle une composition comprenant des acides gras dont plus de 60% sont en C12, C13, C14, C15, C16, C17, C18, C19 et/ou C20, est introduite dans un bassin ou réacteur de traitement des eaux usées, comme substrat pour les microorganismes dénitrifiants.

### Description des figures

La figure 1 (Fig. 1) représente l'évolution des concentrations en azote total (mg/l) en fonction du temps lors d'un procédé de dénitrification, avec des substrats différents pour les microorganismes dénitrifiants : eau, acétate ou une composition selon l'invention.

La figure 2 (Fig. 2) représente l'évolution des concentrations en nitrate (mg/l) en fonction du temps lors d'un procédé de dénitrification, avec des substrats différents pour les microorganismes dénitrifiants : eau, acétate ou une composition selon l'invention.

La figure 3 (Fig. 3) représente l'évolution des concentrations en nitrite (mg/1) en fonction du temps lors d'un procédé de dénitrification, avec des substrats différents pour les microorganismes dénitrifiants : eau, acétate ou une composition selon l'invention.

### Description détaillée de l'invention

La présente invention concerne l'utilisation d'une composition comprenant des acides gras à longues chaînes carbonées (C12 à C20), comme substrat pour microorganismes dénitrifiants.

Ladite composition comprend des acides gras à une concentration pouvant varier de 0,01% à 100% par rapport au poids total de ladite composition, en passant par toutes les valeurs possibles entre ces deux extrêmes.

De façon avantageuse, les acide gras présents dans la composition représentent plus de 30% environ du poids total des MEH (matières extractibles à l'hexane), de préférence plus de 50%, 60%, ou plus de 70% environ, voire plus de 80%, 90%, 95%, 96%, 97% environ du poids total des MEH.

Un objet de la présente invention est plus particulièrement l'utilisation d'une composition substrat pour des microorganismes dénitrifiants comprenant des acides gras à longues chaînes carbonées (C12 à C20), notamment l'acide palmitique, l'acide stéarique, l'acide oléique et/ou l'acide arachidique.

Un objet de la présente invention est plus particulièrement l'utilisation d'une composition comprenant des acides gras dont plus de 60% environ sont en C12, C13, C14, C15, C16, C17, C18, C19 et/ou C20, saturés ou insaturés, comme substrat pour microorganismes dénitrifiants.

Selon un mode préféré de l'invention, la composition utilisée comme substrat pour microorganismes dénitrifiants comprend des matières extractibles à l'hexane (MEH) dont plus de 60% environ, ou plus de 65% environ, ou plus de 70% environ, ou plus de 75% environ, ou de préférence plus de 80% environ, ou plus de 81% environ, ou plus de 82% environ, ou plus de 83% environ, ou plus de 84% environ, ou plus de 85% anviron, ou plus de 86% environ, ou plus de 87% environ, ou plus de 88% environ, ou plus de 89% environ, ou plus de 90% environ sont des acides gras à longues chaînes carbonées (C12 à C20), notamment l'acide palmitique, l'acide stéarique, l'acide oléique et/ou l'acide arachidique.

Le terme « environ », quand il se réfère aux pourcentages en acides gras, signifie « plus ou moins 1% ».

Selon un mode préféré de l'invention, la composition utilisée comme substrat pour microorganismes dénitrifiants comprend des acides gras dont plus de 60% environ, ou plus de 65% environ, ou plus de 70% environ, ou plus de 75% environ, ou de préférence plus de 80% environ, ou plus de 81% environ, ou plus de 82% environ, ou plus de 83% environ, ou plus de 84% environ, ou plus de 85% environ, ou plus de 86% environ, ou plus de 87% environ, ou plus de 88% environ, ou plus de 89% environ, ou plus de 90% environ sont des acides gras à longues chaînes carbonées, saturés ou insaturés, de C12 à C20, notamment l'acide palmitique, l'acide stéarique, l'acide oléique et/ou l'acide arachidique.

Selon un mode préféré de l'invention, la composition utilisée comprend des acides gras saturés ou insaturés, dont plus de 80% sont en C14, C16, C18, et/ou C20, notamment l'acide palmitique, l'acide stéarique, l'acide oléique et/ou l'acide arachidique.

Dans une composition selon l'invention, l'acide palmitique peut représenter environ 20% à environ 45% des acides gras totaux, de préférence environ 30% à 40% des acides gras totaux.

Dans une composition selon l'invention, l'acide stéarique peut représenter environ 25% à environ 60% des acides gras totaux, de préférence environ 30% à environ 45% des acides gras totaux.

Dans une composition selon l'invention, l'acide oléique peut représenter environ 5% à environ 25% des acides gras totaux, de préférence environ 10% à environ 20% des acides gras totaux.

Dans une composition selon l'invention, l'acide arachidique peut représenter jusqu'à environ 25% des acides gras totaux, de préférence jusqu'à environ 15% des acides gras totaux.

Toute combinaison de deux, trois ou quatre des acides palmitique, stéarique, oléique et arachidique, dans l'une quelconque des proportions mentionnées, est également envisagée dans le cadre de la présente invention.

Un objet de la présente invention est également une composition, substrat pour microorganismes dénitrifiants, comprenant environ 20% à environ 45% d'acide palmitique, de préférence environ 30% à 40% d'acide palmitique, environ 25% à environ 60% d'acide stéarique, de préférence environ 30% à environ 45% d'acide stéarique, et/ou environ 5% à environ 25% d'acide oléique, de préférence environ 10% à environ 20% d'acide oléique, par rapport à la totalité des acides gras présents dans la composition.

Une composition selon l'invention peut également comprendre jusqu'à environ 10% d'acide myristique, de préférence jusqu'à environ 5% d'acide myristique, et/ou jusqu'à environ 25% d'acide arachidique, de préférence jusqu'à environ 15% d'acide arachidique, par rapport à la totalité des acides gras présents dans la composition.

Une composition préférée de l'invention comprend environ 30% à 40% d'acide palmitique, environ 30% à environ 45% d'acide stéarique, et environ 10% à environ 20% d'acide oléique, mais également, et optionnellement, jusqu'à environ 5% d'acide myristique et/ou jusqu'à environ 15% d'acide arachidique, par rapport à la totalité des acides gras présents dans la composition.

Une composition selon l'invention comprend des acides gras à une concentration pouvant varier de 0,01% à 100% par rapport au poids total de ladite composition, en passant par toutes les valeurs possibles entre ces deux extrêmes.

De façon avantageuse, les acide gras présents dans la composition représentent plus de 30% environ du poids total des MEH (matières extractibles à l'hexane), de préférence plus de 50%, 60%, ou plus de 70% environ, voire plus de 80%, 90%, 95%, 96%, 97% environ du poids total des MEH.

Une composition selon la présente invention peut également comprendre des correcteurs de pH, des vitamines, du phosphore et/ou autres minéraux, et/ou toutes substances, en particulier inorganiques, favorisant le développement bactérien.

Une composition selon l'invention peut servir de source externe de carbone. Autrement dit, elle peut être préparée en mélangeant des huiles ou autres matières grasses d'origine végétale ou animale pour être utilisée comme substrat pour des microorganismes dénitrifiants.

Elle peut par ailleurs provenir du traitement (ou prétraitement) des eaux usées.

En effet, selon le schéma classique, les matières grasses sont séparées des eaux usées en tête de station d'épuration par une étape de dégraissage/déshuilage. Les matières grasses ainsi récupérées peuvent être envoyées en décharge ou dans un incinérateur, ou peuvent être traitées (généralement sur le site de la station d'épuration).

Ce traitement est effectué dans le but de libérer les longues chaînes d'acides gras contenues dans les lipides puis d'obtenir des acides gras volatiles (C2 à C5) connus comme étant facilement biodégradables.

Ce traitement peut s'effectuer chimiquement, en utilisant des bases fortes, notamment la soude (NaOH), ou par vois enzymatique. Les enzymes impliquées peuvent être ajoutées au milieu ou peuvent provenir de microorganismes présents dans le milieu.

Il peut également s'effectuer dans un réacteur biologique, aéré, en présence d'enzymes, de bactéries utilisées pour métaboliser les acides gras, notamment les bactéries du genre Bacillus, d'agents dispersants, etc. Dans ce cas, le produit obtenu est renvoyé en tête de station afin que les matières grasses insuffisamment dégradées soient à nouveau séparées des eaux usées et retraitées.

Dans le cadre de la présente invention, ce (pré-) traitement n'a pas pour objectif d'obtenir des acides gras à courtes chaînes mais au contraire d'écourter le séjour du milieu à traiter ou d'économiser les moyens mis en oeuvre pour l'aération de façon à obtenir une composition comprenant des acides gras dont plus de 60% sont des acides gras, saturés ou insaturés, à longues chaînes : C12 à C20.

Ce traitement rapide permet de réduire le temps de traitement et le dimensionnement des installations.

Ainsi, l'invention concerne un procédé de préparation d'une composition selon l'invention comprenant les étapes suivantes :
- séparation ou extraction des graisses contenues dans les eaux usées domestiques ou industrielles,
- traitement desdites graisses par des lipases.

Les lipases peuvent être d'origine bactérienne, fongique, animale ou végétale et peuvent être également de synthèse. Toutes enzymes lipolytiques sont envisagées, individuellement ou en mélange, notamment celles du type lipase, phospholipase ou estérase.

L'étape de traitement des graisses peut s'effectuer avec un système complexe comprenant plusieurs enzymes, notamment des lipases, des amylases, des protéases, des cellulases, des microorganismes, par exemple des bactéries du genre *Bacillus* connues pour leur capacité à dégrader les acides gras, des dispersants, notamment des dispersants phosphatés, de l'urée, etc.

Un des avantages de ce procédé de préparation d'une composition selon l'invention réside dans le fait qu'un déchet, la graisse, peut être valorisé comme source de carbone, et peut l'être sur le site de la station d'épuration, après traitement enzymatique et/ou dans un bioréacteur.

Une composition obtenue à l'issue du procédé de l'invention comprend des acides gras à une concentration pouvant varier de 0,01% à 10% par rapport au poids total de ladite composition, avantageusement de 0,05% à 5%, plus particulièrement de 0,09% à 2%.

De façon avantageuse, les acide gras présents dans la composition représentent plus de 30% environ du poids total des MEH (matières extractibles à l'hexane), de préférence plus de 50%, 60%, ou plus de 70% environ, voire plus de 80%, 90%, 95%, 96%, 97% environ du poids total des MEH.

Cette composition - substrat pour microorganismes dénitrifiants peut être utilisée plus particulièrement dans les bassins ou réacteurs des stations d'épuration, dans le traitement des eaux usées.

L'ajout d'une composition selon l'invention dans une eau chargée en azote, en présence de microorganismes dénitrifiants, permet une diminution des teneurs en azote total, en nitrite et en nitrate (respectivement Nₜₒₜₐₗ, NO₂⁻ et NO₃⁻) comparable à celles observées lors de l'ajout d'acétate.

L'invention concerne également un procédé de dénitrification (nitrification - dénitrification) comprenant l'ajout dans un milieu aqueux chargé en azote (présente sous différentes formes, notamment ammoniac, protéines, acides aminés, etc.), en présence de microorganismes dénitrifiants, d'une composition comprenant des acides gras à longues chaînes carbonées (C12 à C20), comme substrat pour lesdits microorganismes dénitrifiants.

Un procédé de dénitrification selon l'invention comprend une étape d'introduction dans un milieu aqueux chargé en azote, en présence de microorganismes dénitrifiants, d'une composition comprenant des acides gras à longues chaînes carbonées (C12 à C20), notamment l'acide palmitique, l'acide stéarique, l'acide oléique et/ou l'acide arachidique.

Ladite composition comprend plus particulièrement des acides gras dont plus de 60% environ, ou de préférence plus de 80% environ, ou plus de 90% environ sont en C12, C13, C14, C15, C16, C17, C18, C19 et/ou C20, saturés ou insaturés.

Selon un mode préféré de l'invention, la composition utilisée dans un procédé de dénitrification selon l'invention comprend des acides gras dont plus de 80% environ sont en C14, C16, C18 et/ou C20, saturés ou insaturés, et en particulier l'acide palmitique, l'acide stéarique, l'acide oléique et/ou l'acide arachidique.

Selon un mode préféré de l'invention, la composition utilisée dans un procédé de dénitrification selon l'invention comprend des matières extractibles à l'hexane (MEH) dont plus de 60% environ, ou plus de 65% environ, ou plus de 70% environ, ou plus de 75% environ, ou de préférence plus de 80% environ, ou plus de 81% environ, ou plus de 82% environ, ou plus de 83% environ, ou plus de 84% environ, ou plus de 85% environ, ou plus de 86% environ, ou plus de 87% environ, ou plus de 88% environ, ou plus de 89% environ, ou plus de 90% environ sont des acides gras à longues chaînes carbonées (C12 à C20), notamment l'acide palmitique, l'acide stéarique, l'acide oléique et/ou l'acide arachidique.

Selon un mode préféré de l'invention, la composition utilisée dans un procédé de dénitrification selon l'invention comprend des acides gras dont plus de 60% environ, ou plus de 65% environ, ou plus de 70% environ, ou plus de 75% environ, ou de préférence plus de 80% environ, ou plus de 81% environ, ou plus de 82% environ, ou plus de 83% environ, ou plus de 84% environ, ou plus de 85% environ, ou plus de 86% environ, ou plus de 87% environ, ou plus de 88% environ, ou plus de 89% environ, ou plus de 90% environ sont des acides gras à longues chaînes carbonées, saturés ou insaturés, de C12 à C20, notamment l'acide palmitique, l'acide stéarique, l'acide oléique et/ou l'acide arachidique.

L'acide palmitique peut représenter environ 20% à environ 45% des acides gras totaux, de préférence environ 30% à 40% des acides gras totaux.

L'acide stéarique peut représenter environ 25% à environ 60% des acides gras totaux, de préférence environ 30% à environ 45% des acides gras totaux.

L'acide oléique peut représenter environ 5% à environ 25% des acides gras totaux, de préférence environ 10% à environ 20% des acides gras totaux.

L'acide arachidique peut représenter jusqu'à environ 25% des acides gras totaux, de préférence jusqu'à environ 15% des acides gras totaux.

Toute combinaison de deux, trois ou quatre des acides palmitique, stéarique, oléique et arachidique, dans l'une quelconque des proportions mentionnées, est également envisagée dans le cadre de la présente invention.

Dans un procédé de dénitrification selon l'invention, la composition - substrat pour microorganismes dénitrifiants peut comprendre environ 20% à environ 45% d'acide palmitique, de préférence environ 30% à 40% d'acide palmitique, environ 25% à environ 60% d'acide stéarique, de préférence environ 30% à environ 45% d'acide stéarique, et environ 5% à environ 25% d'acide oléique, de préférence environ 10% à environ 20% d'acide oléique, par rapport à la totalité des acides gras présents dans la composition. Elle peut également comprendre jusqu'à environ 10% d'acide myristique, de préférence jusqu'à environ 5% d'acide myristique, et/ou jusqu'à environ 25% d'acide arachidique, de préférence jusqu'à environ 15% d'acide arachidique, par rapport à la totalité des acides gras présents dans la composition.

Les microorganismes dénitrifiants dans un procédé selon l'invention peuvent être des bactéries hétérotrophes et dans une moindre mesure des bactéries autotrophes, et sont aérobies facultatives, anaérobies facultatives ou anaérobies. Elles sont capables d'utiliser les ions nitrates et nitrites comme accepteur final de leurs électrons à la place de l'oxygène quand ce dernier est absent du milieu. Elles réduisent ainsi ces ions en azote gazeux qui retourne dans l'atmosphère.

Lesdits bactéries peuvent être de genres ou espèces différentes et peuvent être présentes sous forme de consortium de deux, trois ou plus desdites bactéries. Le genre dominant est *Pseudomonas,* mais d'autres bactéries peuvent également être citées, notamment Agrobacterium *radiobacter, Agrobacterium tumefaciens, Comamonas testoteroni, Alcaligenes faecalis, Alcaligenes denitrificans, Cytophage johnsonae, Aquaspirillium itersonii, Chromobacterium violaceum, Roseobacter denitrificans, Pseudomonas fluorescens.*

Sont donnés ci-après, à titre non limitatifs, des exemples de compositions et de mise en oeuvre du procédé de dénitrification selon l'invention.

### EXAMPLE

### Exemple 1.

L'expérience décrite ci-après permet de comparer la cinétique de dénitrification d'eaux usées sans ajout de carbone assimilable, avec ajout d'acétate ou avec ajout d'une composition telle que décrite précédement.

Le protocole est repris du « Mémento technique de l'eau » (Degrémont, 1989). On mesure l'évolution des teneurs en N total, N-NO₃ et N-NO₂ pour estimer l'efficience du processus de dénitrification.

Trois flacons de 2 1 chacun sont préparés.

L'inoculum est constitué de la liqueur prélevée dans l'aérateur de la STEP de Wavre (centre de la Belgique), comprenant un consortium de microorganismes dénitrifiants. Chaque flacon reçoit 650 ml de liqueur.

La teneur en NO₃ de cette liqueur est mesurée et ajustée avant le lancement de l'expérience à une concentration de départ de 50 mg/l par l'ajout de KNO₃.

Un des trois flacon reçoit en plus 0,6 1 d'une composition telle que décrite précédement. Le deuxième flacon reçoit une charge identique en acétate dissous dans l'eau (solution portée à 0,6 1). Le troisième flacon servant de blanc reçoit 0,6 1 d'eau.

Un bullage à l'azote est réalisé durant 20 minutes pour éliminer l'oxygène. Les trois flacons sont fermés hermétiquement. Un tube plonge dans la liqueur via le bouchon et est relié à une pompe péristaltique pour effectuer les prélèvements.

Les flacons placés dans un incubateur à 30°C.

A intervalle précis (30 min, 1h, 3h, 6h, 9h et 24h), des prélèvements sont effectués et les teneurs en Nₜₒₜₐₗ, N-NO₂ et N-NO₃ sont mesurées grâce à des kits Merck nécessitant une mesure spectrophotométrique.

L'acétate est réputée être une forme de carbone très facilement assimilable par les microorganismes. En plus de la comparaison avec un traitement témoin, la comparaison entre les résultats du "traitement COMPOSITION" et ceux du "traitement acétate" donne donc une bonne indication de l'efficacité de l'utilisation d'une composition selon l'invention comme source de carbone.

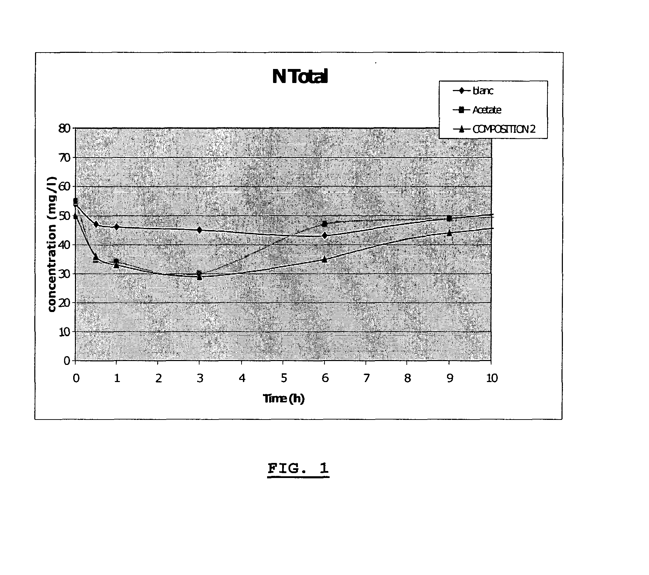

Comme le représente la figure 1, les concentrations en Nₜₒₜₐₗ du "traitement COMPOSITION" et du "traitement acétate" diminuent de 28% et 37,4% respectivement durant la première demi-heure de traitement. La concentration du blanc diminue, elle, de 13% sur le même intervalle. Cette diminution se prolonge durant les trois premières heures pour atteindre 45,5% pour le "traitement acétate", 42% pour "traitement COMPOSITION" et 16,7% pour le blanc (après 6h).

Le "traitement acétate" montre logiquement une grande différence par rapport au témoin et est le plus efficace. En comparaison, le "traitement COMPOSITION" montre également une forte réduction de la teneur en azote total, avec une tendance très semblable à celle du "traitement acétate".

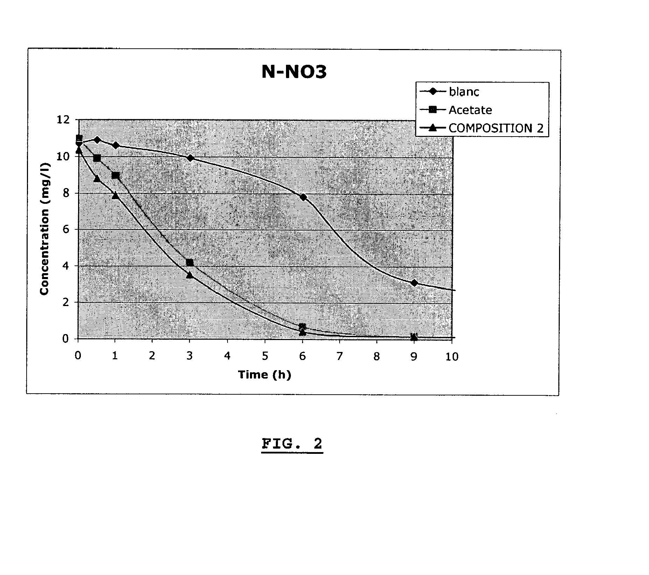

Quant à la diminution des concentrations en nitrates, comme l'indique la figure 2, elle est très similaire pour les deux traitements "COMPOSITION" et "acétate". Elle atteint respectivement 96,1% et 95,8% après 6 heures. Cette diminution n'est que de 28,3% pour le blanc sur le même intervalle.

En fait, le "traitement COMPOSITION" est légèrement plus performant que le "traitement acétate" puisque durant la première heure, la diminution de la concentration en nitrate est respectivement de 23,9% et 18,5%.

A nouveau, les traitements "COMPOSITION" et "acétate" sont semblables et montrent une grande différence par rapport au témoin.

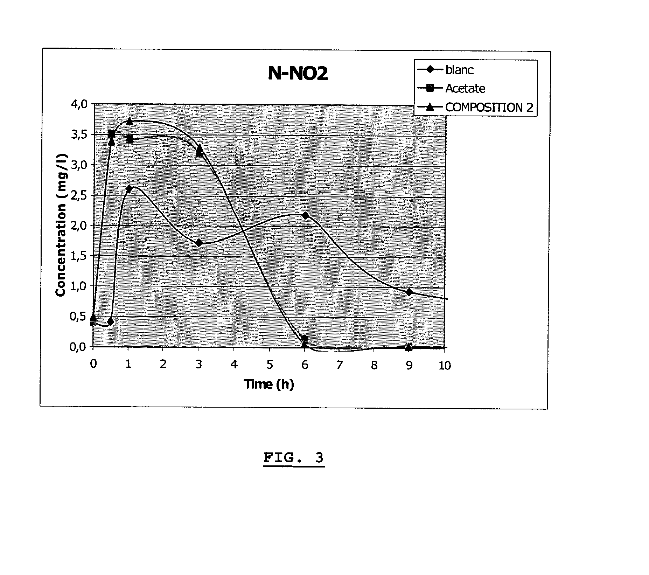

Enfin, concernant les concentrations en nitrites, et comme l'indique la figure 3, elles augmentent fortement pour les traitements "COMPOSITION" et "acétate" durant la première demi-heure. Une diminution très importante des concentrations de ces traitements survient ensuite entre la 3^{e} et la 6^{e} heure, 98,4% et 97% respectivement. Le blanc montre une courbe très différente. La brusque augmentation survient entre 1/2h et 1 heure et la diminution de la concentration ne commence qu'à partir de la 6^{e} heure avec une pente moins importante.

Encore une fois, les traitements "COMPOSITION" et "acétate" sont semblables et montrent une diminution des concentrations en nitrite bien plus rapide et importante que le traitement témoin.

L'expérience refaite en faisant varier tour à tour la composition selon l'invention et la liqueur (prélèvements d'autres stations d'épuration), donne des résultats très similaires.

En effet, on observe pour les trois concentrations suivies que l'ajout de carbone, qu'il soit sous forme d'acétate ou sous la forme d'une composition selon l'invention, favorise fortement le processus de dénitrification. Ce phénomène, bien connu et pris en compte dans les processus de dénitrification en ce qui concerne l'acétate, montre bien qu'une composition selon l'invention peut être considérée comme une source de carbone facilement assimilable et utilisable en dénitrification.

## Revendications

1. Utilisation d'une composition comprenant des acides gras saturés ou insaturés, dont plus de 60% sont en C12, C13, C14, C15, C16, C17, C18, C19 et/ou C20, comme substrat pour microorganismes dénitrifiants.

2. Utilisation d'une composition selon la revendication 1 comprenant des acides gras saturés ou insaturés, dont plus de 60% sont en C14, C16, C18, et/ou C20.

3. Utilisation d'une composition selon la revendication 1 ou 2 comprenant 20% à 40% d'acide palmitique, 30% à 60% d'acide stéarique, et/ou 5% à 25% d'acide oléique, par rapport à la totalité des acides gras présents dans la composition, comme substrat pour microorganismes dénitrifiants.

4. Utilisation d'une composition selon la revendication 2 ou 3 comprenant également jusqu'à 10% d'acide myristique et/ou jusqu'à 25% d'acide arachidique par rapport à la totalité des acides gras présents dans la composition.

5. Composition pour microorganismes dénitrifiants comprenant 20% à 40% d'acide palmitique, 30% à 60% d'acide stéarique et 5% à 25% d'acide oléique par rapport à la totalité des acides gras présents dans la composition.

6. Composition selon la revendication 5 comprenant également jusqu'à 10% d'acide myristique et/ou jusqu'à 25% d'acide arachidique, par rapport à la totalité des acides gras présents dans la composition.

7. Procédé de dénitrification comprenant une étape d'introduction, dans un milieu azoté, en présence de microorganismes dénitrifiants, d'une composition comprenant des acides gras dont plus de 60% sont en C12, C13, C14, C15, C16, C17, C18, C19 et/ou C20, comme substrat pour lesdits microorganismes dénitrifiants.

8. Procédé de dénitrification selon la revendication 7 dans lequel ladite composition comprend des acides gras, saturés ou insaturés, dont plus de 60% environ sont en C14, C16, C18 et/ou C20.

9. Procédé de traitement des eaux usées domestiques ou industrielles comprenant une étape de dénitrification au cours de laquelle une composition comprenant des acides gras dont plus de 60% sont en C12, C13, C14, C15, C16, C17, C18, C19 et/ou C20, est introduite dans un milieu azoté, en présence de microorganismes dénitrifiants.

10. Procédé de traitement des eaux usées selon la revendication 9 dans lequel ladite composition comprend des acides gras, saturés ou insaturés, dont plus de 60% environ sont en C14, C16, C18 et/ou C20.

11. Procédé de préparation d'une composition selon la revendication 5 comprenant les étapes de :
- séparation ou extraction des graisses contenues dans les eaux usées domestiques ou industrielles,
- traitement desdites graisses par des lipases.

12. Procédé selon la revendication 11 dans lequel le traitement des graisses s'effectue avec un système enzymatique comprenant outre les lipases, des amylases, des protéases et des cellulases, avec des microorganismes, avec des dispersants, dont des dispersants phosphatés, et/ou avec de l'urée.
